Europäisches Patentamt

European Patent Office

Office européen des brevets

(19)

(11) Veröffentlichungsnummer: **0 003 595**
B1

(12) # EUROPÄISCHE PATENTSCHRIFT

(45) Veröffentlichungstag der Patentschrift:
14.10.81

(21) Anmeldenummer: 79100352.8

(22) Anmeldetag: 07.02.79

(51) Int. Cl.³: **A 61 B 10/00,** G 01 S 7/56,
H 04 N 5/21

(54) Verfahren und Vorrichtung zur Gewinnung und Aufzeichnung von Ultraschall-Schnittbildern.

(30) Priorität: 14.02.78 DE 2806176

(43) Veröffentlichungstag der Anmeldung:
22.08.79 Patentblatt 79/17

(45) Bekanntmachung des Hinweises auf die Patenterteilung:
14.10.81 Patentblatt 81/41

(84) Benannte Vertragsstaaten:
DE FR GB NL

(56) Entgegenhaltungen:
DE-B-2 711 611
US-A-3 725 928
US-A-3 757 156
US-A-3 975 704
US-A-3 979 555
US-A-3 983 320
IBM TECHNICAL DISCLOSURE BULLETIN,
Band 21, Nr. 6, November 1978,
New York, USA,
WINTER: »Two-dimensional image interpolation«, Seiten 2509—2510

(73) Patentinhaber: **SIEMENS AKTIENGESELLSCHAFT Berlin
und München, Postfach 22 02 61, D-8000 München 22 (DE)**

(72) Erfinder: **Hassler, Dieter, Flurweg 3, D-8521 Uttenreuth
(DE)**

## Verfahren und Vorrichtung zur Gewinnung und Aufzeichnung von Ultraschall-Schnittbildern

Die Erfindung bezieht sich auf ein Verfahren und eine Vorrichtung zur Gewinnung und Aufzeichnung von Ultraschall-Schnittbildern, wobei ein Untersuchungsobjekt mit Ultraschall-Sendesignalen zeilenweise abgetastet wird und die anfallenden Echosignale entsprechend zeilenweise auf dem Aufzeichnungsträger einer Bildaufzeichnungsvorrichtung zum Echo-Sichtbild aufgezeichnet werden. Unter Aufzeichnungsträger einer Bildaufzeichnungsvorrichtung ist dabei insbesondere der Bildschirm einer Oszillographenröhre zu verstehen. Darunter fallen jedoch aber auch im weitesten Sinne die Registrierträger von Schreibern jeglicher Art, die aufgrund von Strich-, Punkt-, Tröpfchenschreibung od. dgl. auf dem Träger Sichtbilder liefern, die in der Schwärzungs- oder auch Farbstruktur in Abhängigkeit von der Intensität anfallender Echosignale moduliert sind.

Bei bekannten Schnittbild-Aufzeichnungsvorrichtungen tritt der Effekt auf, daß in mittleren und hautnahen Bildbereichen die Dynamik der elektrischen Echosignale wesentlich höher ist als jener Amplitudenumfang, der sich mit Intensitätsmodulation des Schreibstrahles im Sichtbild der Aufzeichnungsvorrichtung wiedergeben läßt. Dadurch geht jedoch wesentliche Information verloren, die zur besseren Bildinterpretation beitragen könnte. Erfolgt beispielsweise die Bildaufzeichnung mit niedriger Verstärkung, so lassen sich bestimmte Strukturen in mittleren und hautnahen Bildbereichen in der äußeren Abgrenzung gut, in den inneren Feinstrukturen schon weniger gut abbilden. Letztere Informationen erhält man, wenn die Verstärkung gesteigert wird. Dabei werden allerdings bereits gut strukturierte Bildbereiche überstrahlt und verlieren somit ihren Detailreichtum bzw. ihre Auflösung. In Anbetracht dieses Nachteils ist eine Bildwiedergabe wünschenswert, die die jeweils diagnostisch wertvollen Bildteile des Aufzeichnungsbildes bei niedriger Verstärkung und jenes bei höherer Verstärkung zu einem einzigen Bild vereinigt.

Eine derartige Vereinigung zu einem Gesamtbild ließe sich zwar mit fotografischem Trick durch sog. Abwedeltechnik erreichen, wo also bei der Herstellung eines Positivbildes im Fotolabor jene Stellen eines idealen Negatives, bei denen eine Überbelichtung zu befürchten ist, für eine gewisse Zeitlang abgedeckt werden (abgewedelt werden). So bleiben Details erhalten, die sonst in den schwarzen Bildpartien untergehen würden. Diese Technik ist jedoch im Vergleich zum erreichten Bildverbesserungsgrad recht aufwendig. Außerdem muß bei einem Sichtbild mit niedriger Verstärkung erwartet werden, daß die Kontur aufreißt, weil die Echos von nicht senkrecht getroffenen Strukturen winkelabhängig sehr rasch kleiner werden, je tangentialer der Schallstrahl auftrifft. Der Praktiker wendet daher im allgemeinen ein anderes Verfahren an, das darin beruht, daß er während der Untersuchung bei unterschiedlichen Applikatorstellungen die Verstärkung ändert und das sich ergebende Sichtbild jeweils abfotografiert. Der anschließende Vergleich der verschiedenen Fotos erlaubt dann die diagnostische Aussage. Solange die Konzentration auf einem beschränkten Bildbereich ruht, ist dieses Vorgehen sicher akzeptabel, da es mit nicht allzu hohen Kosten verbunden ist. Soll jedoch ein Ultraschallschnittbild in seiner Gesamtheit beurteilt werden, so ergeben sich nicht unerhebliche Nachteile. Das menschliche Gehirn als Speicher und Computer wird jetzt wesentlich mehr beansprucht. Die Anforderungen können zwar verringert werden dadurch, daß die Bildeinstellung manuell immer wiederkehrend verändert (gewobbelt) wird, so daß wenigstens der Erinnerungsvorgang unterstützt wird. Dieses Vorgehen ist jedoch lediglich eine Hilfslösung, die zeitraubend ist und insbesondere auch die Dokumentation erschwert.

Weitere Möglichkeiten zur Sichtbildverbesserung ergeben sich dadurch, daß entweder mit Farbcodierung im Sichtbild oder mit Dynamikkompression im Echoempfangssignal gearbeitet wird. Die Farbcodierung führt jedoch leicht zu Mißinterpretationen, da jeder Farbumbruch im Sichtbild vom menschlichen Auge automatisch als Kontur gedeutet wird. Die angedeutete Dynamikkompression ließe sich durch nichtlineare Amplitudenverzerrung, beispielsweise Logarithmierung an einer Diode, vornehmen. Das Problem liegt jedoch darin, daß dann alle Störeffekte, insbesondere auch die unscharfen zeitlichen Begrenzungen des Abtaststrahls einschließlich der Nebenkeulen, stark angehoben werden, so daß speziell die Querauflösung sich verschlechtert.

Aus der Zeitschrift »Fernseh- und Kino-Technik« Nr. 11/1976, Seiten 388 bis 392 bzw. auch aus »nachrichten elektronik« 1, 1977, Seiten 11 und 12 ist nun bereits auf dem Gebiet der Fernsehtechnik ein lokaladaptives Bildverarbeitungsverfahren zum Helligkeitsausgleich von ungleichmäßig ausgeleuchteten Fernsehbildern vorbekannt. Speziell in diesem Falle ergibt sich das Dynamikproblem beispielsweise bei Bildern mit starken Schlagschatten. Bei optimaler Einstellung des hellen Bildteiles sind in der Schattenzone keine Einzelheiten mehr zu erkennen. Dem wird durch Dynamikkompression abgeholfen, die eine Aufhellung des Schattenbereichs ermöglicht. Dazu wird mittels zweitem, unscharf aufgenommenen Fernsehbild die integrale Helligkeit in einem Umfeld des darzustellenden Bildpunkts gemessen und die Helligkeit des Punktes im umgekehrten Verhältnis zum Meßergebnis angehoben. Bei einem sehr dunklen Umfeld bedeutet dies also eine starke, bei einem hellen Umfeld entsprechend eine schwächere Anhebung. Auf diese Weise ergibt sich also eine Reduktion der Bilddynamik ohne Beeinflussung des Detailkontrastes des Bildes.

Aus der US-A 39 83 320 ist es außerdem zur besseren Detailerkennung von Rasterbildern auf dem Bildmonitor bekannt, ein mit den Bildpunkten mitlaufendes abgegrenztes Umfeld aus einer vorgewählten Anzahl von benachbarten Bildelementen zu erfassen. Die Bildelementdaten innerhalb dieses Umfeldes werden dann jeweils abgespeichert und nachfolgend einer Summationseinrichtung zugeführt, um dann mit den so erhaltenen Informationsdaten die einzelnen ursprünglichen Bildpunktsignale zur Verbesserung der Bildqualität entsprechend zu beeinflussen. Da hierbei stets nur die das jeweilige Umfeld zusammensetzenden Bildelemente und nicht ganze Zeilen abgespeichert werden, muß für jeden neuen Bildpunkt das mitwandernde Umfeld neu aufsummiert und dessen an das Umfeld entsprechend angepaßte Intensität neu berechnet werden, was schaltungsmäßig relativ aufwendig ist.

Die Anwendung des soeben beschriebenen Verfahrens würde, bezogen auf ein Ultraschall-Schnittbild, jedoch die Orientierung beim Suchvorgang (z. B. Pankreas, Plazenta) unterstützen, weil die Einstellung der Bildparameter (Verstärkung, Tiefenausgleich) unkritischer ist. Die Lokalisationsdiagnostik würde durch die verbesserte Darstellung gekrümmter Organgrenzen erheblich bereichert. Die Auflösung (Bildschärfe) bliebe bei dieser Art Kompression unangetastet, weil das Amplitudenverhältnis der gewünschten zu den unerwünschten Bildpunkten (von Nebenkeulen) unverändert bleibt. Dazu müßte das Umfeld so groß gewählt werden, daß Echos von Haupt- und Nebenkeulen gleichzeitig erfaßt werden. Dann sorgt das Hauptkeulenecho dafür, daß die Verstärkung der Nebenkeulenechos klein bleibt und diese nicht herausgehoben werden. Erst solche Strukturechos, die in größerem seitlichen Abstand liegen, würden aus relativ dunklem Umfeld durch Verstärkungsanhebung herauspräpariert.

Aufgabe vorliegender Erfindung ist es, das aus der Fernsehtechnik vorbekannte Kompressionsverfahren zur Gewinnung und Aufzeichnung speziell von Ultraschall-Schnittbildern in der Weise umzuwandeln, daß die Kompression bei geringstem technischem Aufwand am elektrischen Ultraschall-Signal vorgenommen wird.

Diese Aufgabe wird erfindungsgemäß dadurch gelöst, daß vor Weiterleitung der Echosignale zur Bildaufzeichnungsvorrichtung im Takt des Echozeilenanfalles in zeitlicher Aufeinanderfolge jeweils eine vorwählbare Anzahl von Zeilen, die vorzugsweise zusammen mit einer aktuellen Zeile die Breite eines Umfeldes für abzubildende Echos festlegen, in eine entsprechend vorgegebene Zahl von Zwischenspeichern einer ersten Speichereinheit eingeschrieben wird, die anschließend mit einem Auslesetakt, der vorzugsweise dem Einlesetakt entspricht, abgetastet werden, und daß die dabei jeweils anfallenden Echosignalinformationen dann einer Auswerteeinrichtung zugeleitet werden zur Auswertung in dem Sinne, daß während einer vorgegebenen Ausschnittszeit, die die sich in Zeilenrichtung erstreckende Tiefe des Umfeldes für abzubildende Echos im Zeilenfeld festlegt, wenigstens näherungsweise ein Mittelwert der Echointensität im Umfeld erfaßt und, gegebenenfalls nach vorheriger Dynamikbegrenzung, an einem Verhältnisbildner ins Verhältnis gesetzt wird zum jeweils abzubildenden Echo, dessen Ausgangssignal das Aufzeichnungssignal für die Aufzeichnungsvorrichtung ist, wobei die Auswerteeinrichtung eine Summiereinrichtung umfaßt, der die an den Zwischenspeichern anfallenden Echosignalinformationen aus in Zeilenrichtung gesehen jeweils gleichen Tiefen jeder Abtastzeile zugeleitet werden und welche diese Signalinformation zusammen mit den zugehörigen aktuellen Werten gleicher Tiefe aufsummiert, und wobei diese Summensignale am Ausgang der Summiereinrichtung in einer zweiten Speichereinheit aufsummiert werden, in der auch die sich auf diese Weise jeweils ergebende Gesamtsumme der Summensignale kontinuierlich abgespeichert wird, und gleichzeitig zur schrittweisen Weitertaktung des Umfeldes für abzubildende Echos im Zeilenfeld auch einer dritten Speichereinheit, vorzugsweise einem Schieberegister, mit einer die sich in Zeilenrichtung erstreckende Tiefe des Umfeldes festlegenden Verzögerungszeit zugeleitet werden, wobei die am Ausgang dieser dritten Speichereinheit verzögert anfallenden Summensignale dem die Gesamtsumme der angefallenen Summensignale repräsentierenden Speichersignal der zweiten Speichereinheit im Sinne einer Subtraktion überlagert werden.

Durch die zeilenmäßige Abspeicherung und die daraus resultierende Umfeldgewinnung durch kumulierende Addition und Subtraktion der gemäß dem vorgegebenen Takt anfallenden Bildpunktdaten wird das Ausgangssignal der zweiten Speichereinheit durch sämtliche, von Anfang an angefallenen Bildpunktdaten repräsentiert, was einen einfachen Schaltungsaufbau ergibt.

Eine Vorrichtung zur Durchführung dieses Verfahrens ist erfindungsgemäß gekennzeichnet durch eine vorgegebene Zahl (z. B. vier) von Zwischenspeichern, in die im Takt des Echozeilenanfalles in zeitlicher Aufeinanderfolge jeweils die gleiche Anzahl (hier ebenfalls vier) von Zeilen einschreibbar ist und von denen jeder eine Zeile speichert, und durch einen den Zwischenspeichern zugeordneten Taktgeber zum Auslesen der zwischengespeicherten Echoinformationen in einem vorgegebenen Auslesetakt, der vorzugsweise dem Einlesetakt entspricht, sowie durch eine Auswerteeinrichtung für die Echosignalinformationen am Ausgang der Zwischenspeicher zum Zwecke der Ermittlung eines Mittelwertes und durch einen Verhältnisbildner zur Bildung des Verhältnisses aus einem abzubildenden Echosignal des Umfeldes und dem gegebenenfalls vorher noch durch einen Dynamikbegrenzer hindurchgeleiteten Mittelwertsignal am Ausgang der Auswerteeinrichtung, wobei die Auswerteeinrichtung eine Summiereinrichtung sowie eine zweite Speichereinheit und eine dritte Speichereinheit umfaßt, von denen die Summiereinrichtung die Echosignalinformationen jeweils gleicher Zeilentiefe am Ausgang der Zwischenspeicher zur Bildung eines Mittelwertes aufsummiert und die zweite Speichereinheit ein Sample-and-Hold-Schaltkreis ist, der alle Summensignale der Summiereinrichtung im Takte des Taktgebers aufsummiert und wobei der Ausgang der Summiereinrichtung direkt mit dem Aufsummiereingang und über die dritte Speichereinheit, vorzugsweise ein Schieberegister, die eine der Tiefe des Umfeldes entsprechende Verzögerungszeit hat, mit einem Subtrahiereingang des Sample-and-Hold-Schaltkreises verbunden ist.

Gemäß der Erfindung wird also für jeweils abzubildende Echos ein Umfeld relativ geringen Umfanges gebildet, das während der Ultraschallabtastung entlang den Zeilen mitwandert. Aus diesem Umfeld läßt sich dann für jedes abzubildende Echo ein Mittelwert der Intensität der dieses Echo unmittelbar umgebenden Echos bilden. Durch anschließende Verhältnisbildung zwischen der Intensität des abzubildenden Echos und der Intensität des diesem Echo zugeordneten Umfeldes ergibt sich dann die erwünschte Lokaladaption bzw. Dynamikkompression. Als abzubildendes Echo kann innerhalb des Umfeldes ein beliebiges Echo gewählt werden. Aus Symmetriegründen empfiehlt es sich jedoch, ein Echo nahe der Mitte oder noch besser das Mittenecho auszuwählen. In einem solchen Falle werden dann die im allgemeinen immer symmetrisch zum Hauptecho liegenden Nebenkeulenechos vom Umfeldbereich mit Sicherheit mitüberdeckt. Innerhalb eines solchen Umfeldes ergibt sich dann eine lineare und ungestauchte Echodarstellung. Wird also in bevorzugter Weise als abzubildendes Echo das Mittenecho des Impulses gewählt, so sollte der Mittelwert der Echointensität im Umfeld, gegebenenfalls nach vorheriger Dynamikbegrenzung, am Verhältnisbildner ins Verhältnis gesetzt werden speziell zu einem Speicherecho, das an den Zwischenspeichern nach einer Verzögerungszeit abgegriffen wird, die einer der halben Zeilenzahl gespeicherten Zeilen des Umfeldes plus der halben Speicherzeit der weiteren Speichereinheit entspricht. Dieses gespeicherte Echo ist dann exakt das gesuchte Mittenecho.

Weitere Vorteile und Einzelheiten der Erfindung ergeben sich aus der nachfolgenden Beschreibung von Ausführungsbeispielen anhand der Zeichnung in Verbindung mit Unteransprüchen.

Es zeigt

Fig. 1 ein erstes Ausführungsbeispiel im Prinzipschaltbild,

Fig. 2 Aufbau und Weitertaktung eines rechteckförmigen Umfeldes für abzubildende Echosignale im Prinzipschema,

Fig. 3 ein Diagramm zu Echosignalverläufen von jeweils fünf aufeinanderfolgenden Zeilen, die die Breite eines Umfeldes festlegen,

Fig. 4 eine zweite Ausführungsform im Prinzipschaltbild, die im Hinblick auf Bauelemente zur Dynamikkompression gegenüber der Ausführungsform der Fig. 1 modifiziert ist,

Fig. 5 eine dritte Ausführungsform mit Kompressor für die Eingangsdynamik und logarithmischem Tiefenausgleich für Ausgangssignale.

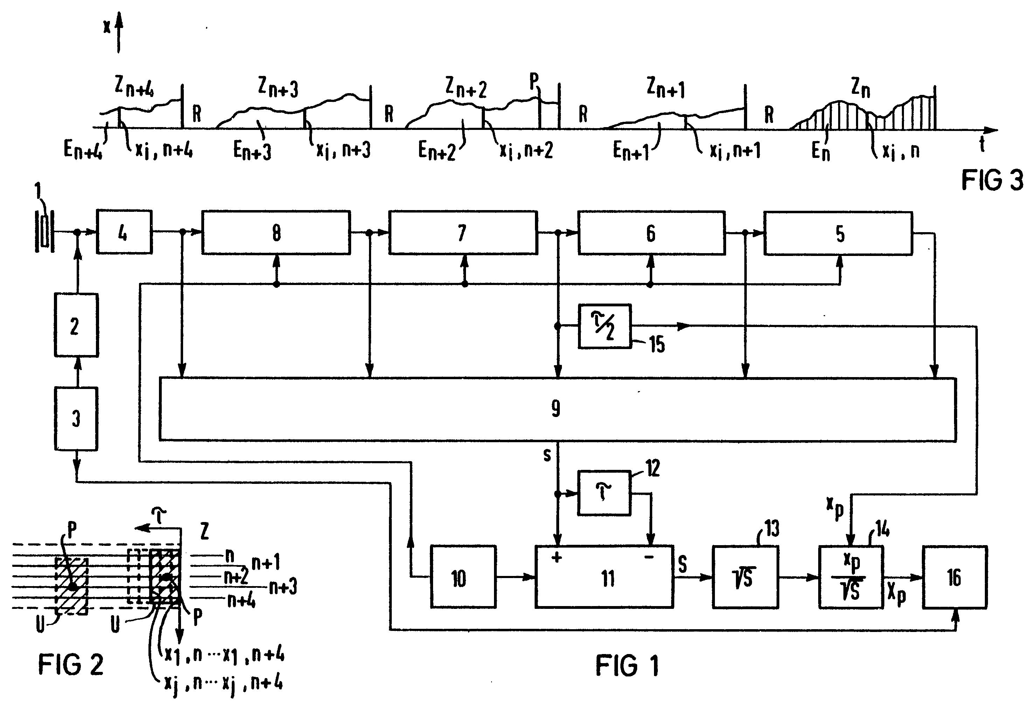

In der Fig. 1 ist mit 1 ein Ultraschallwandler bezeichnet, der ein Untersuchungsobjekt, z. B. menschlichen Körper, zeilenweise abtasten soll. Bei dem rein schematisch dargestellten Wandler 1 kann es sich um einen sog. Rotationswandler mit Paraboloidreflektor, es kann sich ebensogut jedoch auch um einen linear verschiebbaren Wandler bzw. um einen schwenkbaren Wandler für beispielsweise Sector-Scan handeln. Ebensogut kann es sich um einen Wandler eines Compound-Scan-Systems handeln. In sämtlichen dieser Fälle wird der Wandler 1 mit Hochfrequenzsendeimpulsen eines Hochfrequenzimpulserzeugers 2 gespeist. Der Takt der Speisung, d. h. der Takt der Aussendung von Ultraschallimpulsen durch den Wandler 1 in ein Untersuchungsobjekt (nicht dargestellt), wird von den Taktimpulsen eines Taktimpulsgebers 3 vorgegeben. Die aus jeder Ultraschall-Abtastzeile im Untersuchungsobjekt empfangenen Echosignale $x(t)$ werden in der üblichen Weise einem Hochfrequenz-Empfangsverstärker 4 zugeleitet. Im Normalfall würden nun die Ausgangssignale dieses Verstärkers als Echoimpulse direkt zur Aufzeichnung auf eine Bildaufzeichnungsvorrichtung, insbesondere Elektronenstrahlröhre, gegeben werden.

Im vorliegenden Fall wird jedoch gemäß der Erfindung für jedes abzubildende Echo zuerst immer das zugehörige Umfeld gebildet und zur Intensität des abzubildenden Echos ins Verhältnis gesetzt. Erst dieses Verhältnissignal wird dann der Bildaufzeichnungsvorrichtung zur Aufzeichnung zugeleitet.

In der Fig. 2 ist nun passend für das Prinzipschaltbild der Fig. 1 ein Bildausschnitt mit einem aktuell darzustellenden Punkt P in seinem mitwandernden Umfeld U dargestellt. Das Umfeld umfaßt dabei jeweils fünf Zeilen $Z_n$ bis $Z_{n+4}$, die in ihrer Gesamtheit die Breite des Umfeldes U festlegen. Die Tiefe jedes Umfeldes ist mit $\tau$ bezeichnet. Das Umfeld eines jeden darzustellenden Echoimpulses wandert mit dem zugehörigen Echopunkt entlang den Zeilen. Der Takt der Weiterbewegung des Umfeldes ist dabei vorgegeben durch den Empfangs- bzw. Widergabetakt von Echoimpulsen innerhalb einer Zeile. In der Fig. 3, die jeweils vier Vergangenheitszeilen $Z_n$ bis $Z_{n+3}$ zusammen mit der aktuellen Zeile $Z_{n+4}$ zeigt, ist dieser Auflösetakt in der Zeile $Z_n$ mit insgesamt siebzehn Schritten $x_{1,n}$ bis $x_{17,n}$ angedeutet. Ein beliebiger Schritt innerhalb des Zeilenfeldes ist mit $x_{i,n}$ bezeichnet. Entsprechendes geschieht auch mit den anderen Zeilen, wo jeweils immer nur der i-te Schritt $x_{i,n+1}$ für die Zeile $Z_{n+1}$, $x_{i,n+2}$ für die

Zeile $Z_{n+2}$ etc. formal dargestellt ist. Solche (die ersten drei von einer Vielzahl nachfolgender) Schritte finden sich dann auch im schwarzumrandeten Umfeld U für das Echosignal P bei dessen Weitertaktung mit weiterlaufender Zeile. Zwei weitere Umfelder für auf den Punkt P folgende weitere Punkte sind gestrichelt angedeutet. Eine vierte Umfeldposition ist auf der linken Seite der Fig. 2 für einen Punkt P in der Zeile $Z_{n+3}$ schematisch angedeutet.

Die Unterteilung in insgesamt siebzehn Schritte hat lediglich exemplarischen Charakter. In Wirklichkeit beträgt die Punktauflösung einer Zeile mindestens 1 mm. Bei beispielsweise 20 cm-Bildbreite würde dies dann eine Auflösung von mindestens zweihundert Schritten pro Zeile bedingen. In tatsächlicher Realisierung durchläuft dann jedes Umfeld die Zeile also nicht nur in siebzehn, sondern in mindestens insgesamt zweihundert Einzelschritten.

Beim Ausführungsbeispiel der Fig. 1 werden nun die jeweils zeitlich nacheinander am Ausgang des Empfangsverstärkers 4 anfallenden Echozeilen in insgesamt vier Schieberegister 5 bis 8 (vorzugsweise Analog-Schieberegister) eingeschoben. Bezogen auf ein beliebiges Umfeld U mit den Zeilen $Z_n$ bis $Z_{n+4}$ bedeutet dies also, daß die Zeilen $Z_n$ bis $Z_{n+3}$ als Vergangenheitszeilen in der in Fig. 3 dargestellten Form hintereinander liegend in die Schieberegister 5, 6, 7 und 8 eingeschrieben sind. Die Einzelwerte der Zeile $Z_{n+4}$ sind aktuelle Werte, die jeweils unmittelbar anfallen. Der an einem Taktgeber 10 einstellbare Schiebetakt für die Schieberegister 5 bis 8 sowie die Speicherlänge der Einzelregister sind so gewählt, daß der Inhalt einer Echozeile $E_n$, $E_{n+1}$ etc. zusammen mit der zugehörigen Totzeit R für den Zeilenrücksprung exakt in das jeweilige Schieberegister paßt. Auf diese Weise findet sich also die Echoinformation aus gleichen Tiefenbereichen einer jeden Zeile für insgesamt vier Zeilen räumlich nacheinander geordnet in einzelnen Schieberegistern. Durch Abruf dieser Informationen aus den Registern mittels des Taktgebers 10 fallen also immer gleichzeitig an den Registerausgängen gespeicherte Echowerte aus gleichen Tiefen für jede Zeile einschließlich eines zugehörigen aktuellen Tiefenwertes am Eingang des Schieberegisters 8 an. Diese Tiefenwerte jeder Zeile werden nun auf einen Summenbildner 9 gegeben, der ein zugehöriges Summensignal aus zugeordneten Tiefenbereichen einer jeder der fünf Zeilen des mitlaufenden Umfeldes U bildet. Der Schiebetakt des Taktgebers 10 für die Register 5 bis 8 bewirkt also eine Quantisierung der Analog-Echosignale in Werte $x_i$ für unterschiedliche Tiefenlagen $i = 0$ bis j innerhalb des Umfeldes U. Die Summiereinrichtung 9 addiert die jeweils gleichliegenden $x_i$ alle fünf Zeilen des Umfeldstreifens. Es ergeben sich somit Summensignale nach der Beziehung

$$s = \sum_{n}^{n+4} x_{i,n}$$

(n = Zeilennummer).

Die Summation über i erfolgt hingegen mittels Sample-and-Hold-Schaltkreise 11 in Verbindung mit einem Schieberegister 12 mit einer Speicherzeit $\tau$. Der Sample-and-Hold-Schaltkreis 11 addiert alle Summensignale der Summiereinheit 9 im Schiebetakt des Taktgebers 10. Abgezogen von der gespeicherten Gesamtsumme der Summensignale wird jedoch über das Schieberegister 12 jeweils immer der Summenwert der Summierschaltung 9, der vor der Verzögerungszeit des Schieberegisters bereits angefallen war. Auf diese Weise ergibt sich das im x-Schrittakt wandernde Umfeld mit der Tiefenausdehnung $\tau$ nach der Beziehung

$$S = \sum_{i=0}^{i=j} \sum_{n}^{n+4} x_{i,n}$$

Dieser Summenwert S soll nun die Darstellhelligkeit des Punktes P definieren, der in der Mitte des Umfeldes liegt. Das Echosignal zum Punkt P befindet sich an einer Stelle in der Schaltung der Schieberegister 5 bis 8, die speziell einer Verzögerung von zwei gespeicherten Zeilen (einschließlich Austastung R) plus $\tau/2$ entspricht. Das sich hieraus also am Ausgang des Schieberegisters 7 ergebende Signal wird über ein Verzögerungsglied 15 nach $\tau/2$ als Ausgangs-Signal $x_p$ dieses Gliedes dem einen Eingang eines Verhältnisbildners 14 zugeleitet. Dem anderen Eingang des Verhältnisbildners 14 wird das Ausgangssummensignal S des Sample-and-Hold-Schaltkreises 11 nach vorhergehender Dynamikbegrenzung zugeleitet. Als Dynamikbegrenzer dient ein Wurzelbildner 13, der die Wurzel aus der Doppelsumme S bildet. Hierdurch wird die Dynamik des Signals $x_p$ halbiert, wenn das Umfeld dunkel ist. Aus maximal möglichen 80 dB hautnah ergeben sich bei voller Kompression also 40 dB; dies entspricht einem Pegelumfang, der gerade als Helligkeitsmodulation gut darstellbar ist. Je mehr sich jedoch das Umfeld mit Bildpunkten füllt, desto geringer wird die Kompression, da das Nennersignal immer weniger von der Intensität des tatsächlich darzustellenden Echos abhängt.

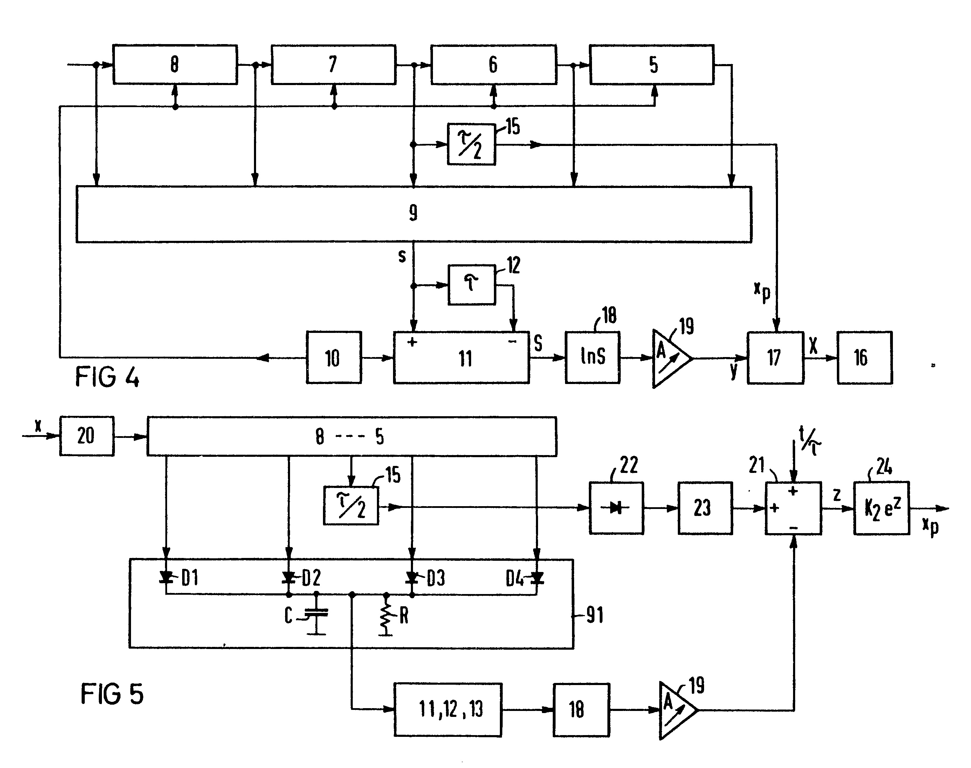

In diesem Zusammenhang kann es auch wünschenswert sein, den Grad der Kompression (Teilungsfaktor der Dynamik im logarithmischen Maß) variabel zu gestalten. Dies kann entweder in Form eines extern zu bedienenden Stellgliedes oder automatisch geschehen. Die Automatik bietet sich an, wenn man die Kompression an den unterschiedlichen Signalstörabstand des Bildes für unterschiedliche Tiefen anpassen will. In diesem Fall muß die Kompression zur Tiefe hin abnehmen. Variabilität der Kompression erfordert die Variation des Exponenten im Nenner des Verhältniswertes. Dies läßt sich jedoch am besten erreichen, wenn logarithmisch gerechnet wird. Ein Ausführungsbeispiel dafür zeigt die Fig. 4.

Zur Verhältnisbildung ist jetzt ein Steuerverstärker 17 (elektrisch variabler Verstärkungsgrad) mit exponentieller Steuerkennlinie

$$v = v_0 \, e^{-Ky}$$

eingesetzt.

Wird nun das Ausgangssummensignal S des Sample-and-Hold-Schaltkreises 11 zuerst einem Logarithmierglied 18 zugeführt und anschließend also der Steuereingang des Steuerverstärkers 17 mit dem Logarithmus des Summensignals beaufschlagt, dann ergibt sich mit

$$y = A \ln S$$

das Ausgangssignal des Steuerverstärkers 17 zu

$$X_p = \frac{x_p \cdot v_0}{S^{KA}} \, .$$

Der Faktor $K \cdot A$ läßt sich nun durch Variation des Verstärkungsfaktors A an einem Verstärker 19 zwischen Logarithmierglied 18 und Steuereingang des Steuerverstärkers 17 beliebig variieren. Für den Fall $K \cdot A = 1/2$ ergibt sich wieder, wie beim Ausführungsbeispiel der Fig. 1, Wurzelbildung und damit eine Halbierung der Dynamik.

In beiden Fällen, d. h. Dynamikkompression eines Wechselsignals durch Direktwurzelbildung oder durch Wurzelbildung aufgrund Exponentenauswahl nach Logarithmierung, lassen sich besonders vorteilhaft dynamikbegrenzte Analoggglieder, z. B. analoge Verzögerungsleitungen, einsetzen, ohne daß Informationsverlust droht.

In den Ausführungsbeispielen der Fig. 1 bis 4 wird den Schieberegistern 5 bis 8 das originale, d. h. das in der Dynamik noch nicht komprimierte, Echosignal angeboten. Selbstverständlich kann auch so vorgegangen werden, daß mittels geeigneten Kompressors das Ursprungssignal gestaucht und dann das gestauchte Signal den Schieberegistern zugeleitet wird. Dieses vorab ohne Umfeldberücksichtigung gestauchte Bild enthält auch weiterhin relativ gut auswertbare Information über helle und dunkle Bereiche. Diese Information kann dann jedoch als Steuerkriterium ausgenutzt werden in dem Sinne, daß überall dort die Verstärkung herabgesetzt bzw. die Stauchung aufgehoben wird, wo bereits das vorab gestauchte Bild hell erscheint. Als Kompressor sollte in diesem Fall ein Wechselstromverstärker dienen, dessen Verstärkungsgrad sich so einstellt, daß die Amplituden des Wechsel- oder Hochfrequenzsignals nach einer vorwählbaren Kennlinie nichtlinear beeinflußt werden. Die Beeinflussung sollte speziell zur Kompression der Dynamik der Amplituden nach Wurzelgesetz oder logarithmischem Gesetz erfolgen. Zur Expansion nach erfolgter Umfeldberücksichtigung kämen als Funktionen die inverse nichtlineare Funktion, im speziellen also die Quadratur oder ein Exponentialgesetz, in Frage. Speziell beim Einsatz eines gesteuerten Verstärkers werden beide Polaritäten des Eingangssignals berücksichtigt. Dies gibt die Möglichkeit, entweder nach einer Doppelweggleichrichtung auf der Seite niedriger Dynamik mit kürzestmöglichen Integrationszeitkonstanten für die Pulsform auszukommen oder nach Polarität getrennt auszuwerten (Reflexion an schallhartem bzw. schallweichem Reflektor). Allerdings müssen Gleichstromwerte mitübertragen bzw. gespeichert werden, da es sich bei der Kompression um eine gesteuerte Nichtlinearität handelt, die aus einem reinen Wechselsignal gleichzeitig Gleichstromanteile neu erzeugt. Mit Hilfe solcher nichtlinearen Verstärker ist es jedoch möglich, von vornherein schon dynamikbegrenzte Bausteine (Schieberegister, Spitzenwertgleichrichter, Dividierer) zwischen einem Kompressor und einem Expander einzubetten. Im Falle dieser Bausteine kann also auf normal käufliche analog arbeitende Bauelemente zurückgegriffen werden.

Bei Kompression des originären Signals, beispielsweise nach der Beziehung

$$x_K = \frac{x}{\sqrt{|x|}}$$

kann die Expansion beispielsweise quadratisch nach der Beziehung

$$x_E = \frac{x_p}{\sqrt{|x_p|}} \cdot \frac{|x_p|}{\sqrt{|x_p|}} = x_p$$

erfolgen.

Der Expander kann dabei vor dem eigentlichen Verhältnisbildner liegen. Lediglich der Verhältnisbildner liegt dann also außerhalb des Schonbereichs eingeengter Dynamik; er erhält also demnach das dekomprimierte Signal hoher Dynamik und muß daher bei dieser Art Signalverarbeitung besonders hochwertig sein. Die Situation wird jedoch vereinfacht, wenn die Reihenfolge von Verhältnisbildner und Expander vertauscht wird; in diesem Falle muß jedoch das Summensignal für den Verhältnisbildner über einen zusätzlichen Wurzelbildner laufen. Nur so erhält man das gleiche Ausgangssignal wie zuvor. Eine besondere Vereinfachung erhält man jedoch bei Modifikation des Ausführungsbeispiels der Fig. 4 im nachbeschriebenen Sinne.

Wird also in diesem Ausführungsbeispiel der Registrierschaltung 5 bis 8 ein Kompressor vorgeschaltet, so können der Logarithmierer 18 und der Steuerverstärker 17 sofort in die Zone eingeschränkter Dynamik hineingenommen werden. Erst auf den Steuerverstärker folgen dann Expander und Gleichrichter. Bei Summenspitzenwertbildung und anschließender zeitlicher Summierung vor Logarithmierung ergibt sich dann am Ausgang des Steuerverstärkers ein Signal mit der Beziehung

$$z = \frac{x_p}{\sqrt{|x_p|}}\, v_0\, e^{-KA \ln \sqrt{|\hat{x}|}} = \frac{v_0 \cdot \dfrac{x_p}{\sqrt{|x_p|}}}{\sqrt{|\hat{x}|}^{\,K \cdot A}}$$

Bei Dekompression im Expander nach der Beziehung

$$z \cdot |z|$$

ergibt sich das dekomprimierte Ausgangssignal

$$x_E = \frac{v_0^2 \cdot \dfrac{x_p}{\sqrt{|x_p|}} \cdot \dfrac{|x_p|}{\sqrt{|x_p|}}}{\sqrt{|\hat{x}|}^{\,2KA}}, \text{ und}$$

mit der Einstellung

$$2\,KA = 1$$

verläuft dann das dekomprimierte Ausgangssignal wiederum nach Wurzelfunktion

$$x_E = v_0^2 \cdot \frac{x_p}{\sqrt{|\hat{x}|}}.$$

Da nach dem Steuerverstärker die Dynamik eingeschränkt bleibt, könnte die Reihenfolge von Expander und Gleichrichter vertauscht werden.

Versetzt man nun jedoch den Gleichrichter noch um eine weitere Stelle nach vorne, was wegen vorausgehender Kompression ohne Schwierigkeiten durchführbar ist, dann ergibt sich die Möglichkeit, die Signalverarbeitung völlig im Logarithmischen ablaufen zu lassen, so daß kein eigenständiger Expander mehr erforderlich ist. Auch die Funktion eines exponentiellen Tiefenausgleichs läßt sich im logarithmischen Verarbeitungsteil mit unterbringen, so daß kein zusätzlicher Tiefenausgleichsverstärker erforderlich ist. Eine in diesem Sinne modifizierte Ausführungsform zeigt die Fig. 5.

In der Fig. 5 ist den Registern 5 bis 8 ein Kompressor 20 vorgeschaltet. Der Kompressor 20 komprimiert die Dynamik des einkommenden Signals x in der oben beschriebenen Weise nach der Funktion

$$x_K = \frac{x}{\sqrt{|x|}}.$$

Am Ausgang des mit Dioden D1 bis D4, Kapazitäten C und Widerständen R gebildeten Summen-Spitzenwertbildners 91 liegt nach zeitlicher Aufsummierung und Wurzelbildung in den Bauelementen 11 bis 13 das Signal

$$\sqrt{|\hat{x}|}\,.$$

Dieses Signal wird im Logarithmierglied 18 zu

$$\ln \sqrt{|\hat{x}|}$$

umgeformt. Das logarithmierte Signal wird über den Verstärker 19 auf den Subtrahiereingang eines Summen-Differenz-Bildners 21 gegeben. Dieser besitzt zusätzlich zwei summierende Eingänge. Dem ersten Summiereingang wird das am Ausgang des Verzögerungsgliedes 15 anfallende Signal

$$x_p/\sqrt{|x_p|}$$

nach Gleichrichtung in einem Gleichrichter 22 und Logarithmierung nach der Beziehung

$$K_1 \ln \sqrt{|x_p|}$$

in einem weiteren Logarithmierglied 23 zugeleitet. Auf den anderen Summiereingang wird eine zeitlineare Rampenfunktion $t/\tau$ für den notwendigen Tiefenausgleich gegeben. Da sich das somit am Ausgang des Differenz-Summen-Bildners 21 bildende Signal z nach der Beziehung $K_2 e^z$ formiert, ergibt sich für das Ausgangssignal des Formgliedes 24 die Beziehung

$$x_p = K_2\, e^{K_1 \ln \sqrt{|x_p|} - A \ln \sqrt{|\hat{x}|} + \frac{t}{\tau}}$$

$$= K_2\, \frac{\sqrt{|x_p|}^{\,K_1}}{\sqrt{|\hat{x}|}^{\,A}} \cdot e^{t/\tau}.$$

Mit

$$K_1 = 2; \quad K_2 = 1; \quad A = 1$$

erhält man schließlich die Form des exponentiellen Tiefenausgleichs zu

$$x_p = \frac{|x_p|}{\sqrt{|\hat{x}|}}\, e^{t/\tau}.$$

Die Logarithmierung braucht nicht wie im vorliegenden Fall in getrennten Blöcken am Ausgang der Verarbeitungsschaltung durchgeführt werden. Sie kann auch im eingangsseitigen Kompressor durchgeführt werden, nämlich dann, wenn der logarithmisch arbeitende Kompressor gleichzeitig Tiefenausgleichsverstärker ist oder wenn eine zeitlineare Rampenfunktion am Kompressor für den notwendigen Tiefenausgleich sorgt.

In sämtlichen Ausführungsbeispielen der Fig.1 bis 5 kommen als Schieberegister bevorzugt Analog-Speicherkettenschaltungen, insbesondere sogenannte CCD-Schaltungen, in Betracht, weil mit diesen eine gute Synchronisation mit der Zeilenfrequenz möglich ist. Ebensogut lassen sich jedoch auch akustische Verzögerungsleitungen einsetzen, wie sie beispielsweise in Farbfernsehgeräten als Pal-Leitungen üblich sind.

## Patentansprüche

1. Verfahren zur Gewinnung und Aufzeichnung von Ultraschall-Schnittbildern, wobei ein Untersuchungsobjekt mit Ultraschall-Sendesignalen zeilenweise abgetastet wird und die anfallenden Echosignale entsprechend zeilenweise auf dem Aufzeichnungsträger einer Bildaufzeichnungsvorrichtung zum Echo-Sichtbild aufgezeichnet werden, dadurch gekennzeichnet, daß vor Weiterleitung der Echosignale ($E_n$ bis $E_{n+4}$) zur Bildaufzeichnungsvorrichtung (16) im Takt des Echozeilenanfalles in zeitlicher Aufeinanderfolge jeweils eine vorwählbare Anzahl (z. B. vier) von Zeilen ($Z_n$ bis $Z_{n+3}$), die vorzugsweise zusammen mit einer aktuellen Zeile ($Z_{n+4}$) die Breite eines Umfeldes (U) für abzubildende Echos (P) festlegen, in eine entsprechend vorgegebene Zahl von Zwischenspeichern (5, 6, 7, 8) einer ersten Speichereinheit eingeschrieben wird, die anschließend mit einem Auslesetakt, der vorzugsweise dem Einlesetakt entspricht, abgetastet werden, und daß die dabei jeweils anfallenden Echosignalinformationen dann einer Auswerteeinrichtung (9 bis 12) zugeleitet werden zur Auswertung in dem Sinne, daß während einer vorgegebenen Ausschnittszeit, die die sich in Zeilenrichtung erstreckende Tiefe ($\tau$) des Umfeldes (U) für abzubildende Echos (P) im Zeilenfeld festlegt, wenigstens näherungsweise ein Mittelwert (S) der Echointensität im Umfeld erfaßt und, gegebenenfalls nach vorheriger Dynamikbegrenzung, an einem Verhältnisbildner (14) ins Verhältnis gesetzt wird zum jeweils abzubildenden Echo ($x_p$), dessen Ausgangssignal ($X_p$) das Aufzeichnungssignal für die Aufzeichnungsvorrichtung (16) ist, wobei die Auswerteeinrichtung eine Summiereinrichtung (9) umfaßt, der die an den Zwischenspeichern (5 bis 8) anfallenden Echosignalinformationen aus in Zeilenrichtung gesehen jeweils gleichen Tiefen jeder Abtastzeile zugeleitet werden und welche diese Signalinformationen zusammen mit den zugehörigen aktuellen Werten gleicher Tiefe aufsummiert, und wobei diese Summensignale (s) am Ausgang der Summiereinrichtung (9) in einer zweiten Speichereinheit (11) aufsummiert werden, in der auch die sich auf diese Weise jeweils ergebende Gesamtsumme der Summensignale (s) kontinuierlich abgespeichert wird, und gleichzeitig zur schrittweisen Weitertaktung des Umfeldes (U) für abzubildende Echos im Zeilenfeld auch einer dritten Speichereinheit (12), vorzugsweise einem Schieberegister, mit einer die sich in Zeilenrichtung erstreckende Tiefe des Umfeldes festlegenden Verzögerungszeit ($\tau$) zugeleitet werden, wobei die am Ausgang dieser dritten Speichereinheit (12) verzögert anfallenden Summensignale dem die Gesamtsumme der angefallenen Summensignale repräsentierenden Speichersignal der zweiten Speichereinheit (11) im Sinne einer Subtraktion überlagert werden.

2. Verfahren nach Anspruch 1, dadurch gekennzeichnet, daß von den Zwischenspeichern (5 bis 8) gespeicherte Echosignalinformationen aus gleichen Tiefen jeder Abtastzeile gleichzeitig abgetastet und gleichzeitig an die Summiereinrichtung (9) zur Aufsummierung weitergeleitet werden.

3. Verfahren nach Anspruch 1 oder 2, dadurch gekennzeichnet, daß der Mittelwert der Echointensität im Umfeld, gegebenenfalls nach vorheriger Dynamikbegrenzung, an dem Verhältnisbildner (14) ins Verhältnis gesetzt wird zu einem Speicherecho ($x_p$), das an den Zwischenspeichern (5 bis 8) nach einer Verzögerungszeit abgegriffen wird, die der halben Zeilenzahl gespeicherter Zeilen des Umfeldes plus der halben Speicherzeit der weiteren Speichereinheit (11) entspricht.

4. Verfahren nach einem der Ansprüche 1 bis 3, dadurch gekennzeichnet, daß anfallende Echoinformationen aus einzelnen Zeilen kontinuierlich in den einzelnen Zeilen zugeordnete und in Serie geschaltete Schieberegister (5 bis 8) als Zwischenspeicher ein- und durch diese hindurchgetaktet werden und daß jeweils die an den Ausgängen eines jeden Schieberegisters einschließlich der jeweils gerade am Eingang des jeweils ersten Schieberegisters anfallenden Echosignalinformationen im Anfalltakt auf die Auswerteeinrichtung (9 bis 12) gegeben werden.

5. Verfahren nach einem der Ansprüche 1 bis 4, dadurch gekennzeichnet, daß das Mittelwertsignal (S) dem Verhältnisbildner (14) zur Dynamikkompression über einen Wurzelbildner (13) zugeleitet wird (Fig. 1).

6. Verfahren nach einem der Ansprüche 1 bis 5, dadurch gekennzeichnet, daß zur Verhältnisbildung (Verhältnisbildner 14) das Mittelwertsignal (S) nach Logarithmierung in einem Logarithmusbildner (18) als Steuersignal (y) dem Steuereingang für den Verstärkungsgrad eines Steuerverstärkers (17) mit einer exponentiellen Steuerkennlinie mit negativem Exponenten ($v_0 \cdot e^{-Ky}$) zugeleitet wird, an dessen Verstärkungseingang das jeweils abzubildende Echosignal anliegt (Fig. 4).

7. Verfahren nach Anspruch 6, dadurch gekennzeichnet, daß das logarithmierte Mittelwertsignal durch einen zwischengeschalteten Signalverstärker (19) im Verstärkungsgrad (A) beliebig einzustellen ist, so daß sich beispielsweise am Ausgang des Steuerverstärkers (17) ein Verhältnissignal ergibt, dessen Nennergröße ($S^{KA}$) sich aus dem eingestellten Verstärkungsgrad (A) bestimmt.

8. Verfahren nach Anspruch 7, dadurch gekennzeichnet, daß bei Einstellung des Exponenten auf den Wert 1/2 wiederum Wurzelabhängigkeit und damit Halbierung der Dynamik erreicht ist.

9. Verfahren nach einem der Ansprüche 1 bis 4, dadurch gekennzeichnet, daß bereits das originäre Echosignal (x) vor Zwischenspeicherung in den Zwischenspeichern (5 bis 8) zwecks Dynamikbegrenzung komprimiert wird (Fig. 5).

10. Verfahren nach Anspruch 9, dadurch gekennzeichnet, daß die Kompression nach Wurzel- oder Logarithmusfunktion erfolgt.

11. Verfahren nach Anspruch 9 oder 10, dadurch gekennzeichnet, daß abzubildende Echosignale vor Verhältnisbildung oder das Verhältnissignal selbst mittels Expander dekomprimiert werden.

12. Verfahren nach Anspruch 11, dadurch gekennzeichnet, daß die Expansion nach quadratischem oder logarithmischem Gesetz erfolgt.

13. Verfahren nach einem der Ansprüche 9 bis 12, dadurch gekennzeichnet, daß die jeweils abzubildenden Echosignale $(x_p)$ nach Gleichrichtung in einem Gleichrichter (22) mittels Logarithmierglied (23) logarithmiert und das logarithmische Signal einem ersten Summiereingang eines Summen-Differenz-Bildners (21) zugeleitet wird, dessen zweiter Summiereingang mit einer Rampenfunktion $(t/\tau)$ und dessen Subtrahiereingang mit dem logarithmischen Summensignal für die Verhältnisbildung beaufschlagt sind (Fig. 5).

14. Vorrichtung zur Durchführung des Verfahrens nach einem oder mehreren der Ansprüche 1 bis 13, gekennzeichnet durch eine vorgegebene Zahl (z. B. vier) von Zwischenspeichern (5 bis 8), in die im Takt des Echozeilenanfalles in zeitlicher Aufeinanderfolge jeweils die gleiche Anzahl (hier ebenfalls vier) von Zeilen einschreibbar ist und von denen jeder eine Zeile speichert, und durch einen den Zwischenspeichern (5 bis 8) zugeordneten Taktgeber (10) zum Auslesen der zwischengespeicherten Echoinformationen in einem vorgegebenen Auslesetakt, der vorzugsweise dem Einlesetakt entspricht, sowie durch eine Auswerteeinrichtung (9 bis 12) für die Echosignalinformationen am Ausgang der Zwischenspeicher zum Zwecke der Ermittlung eines Mittelwertes (S) und durch einen Verhältnisbildner (14) zur Bildung des Verhältnisses aus einem abzubildenden Echosignal des Umfeldes und dem gegebenenfalls vorher noch durch einen Dynamikbegrenzer (13) hindurchgeleiteten Mittelwertsignal am Ausgang der Auswerteeinrichtung, wobei die Auswerteeinrichtung eine Summiereinrichtung (9) sowie eine zweite Speichereinheit (11) und eine dritte Speichereinheit (12) umfaßt, von denen die Summiereinrichtung (9) die Echosignalinformationen jeweils gleicher Zeilentiefe am Ausgang der Zwischenspeicher (5 bis 8) zur Bildung eines Mittelwertes aufsummiert und die zweite Speichereinheit (11) ein Sample-and-Hold-Schaltkreis ist, der alle Summensignale der Summiereinheit (9) im Takte des Taktgebers (10) aufsummiert, und wobei der Ausgang der Summiereinrichtung (9) direkt mit dem Aufsummiereingang und über die dritte Speichereinheit (12), vorzugsweise ein Schieberegister, die eine der Tiefe des Umfeldes entsprechende Verzögerungszeit $(\tau)$ hat, mit einem Subtrahiereingang des Sample-and-Hold-Schaltkreises (11) verbunden ist.

15. Vorrichtung nach Anspruch 14, gekennzeichnet durch Spitzendetektoren (91) in der Auswerteeinrichtung zur Erfassung einzelner Echointensitäten.

16. Vorrichtung nach Anspruch 14 oder 15, gekennzeichnet durch eine Serienschaltung aus Analog-Schieberegistern (5 bis 8) als Zwischenspeicher, die ein- bzw. ausgangsseitig mit Eingängen der Auswerteeinrichtung (9 bis 12) verbunden sind.

17. Vorrichtung nach einem der Ansprüche 14 bis 16, gekennzeichnet durch einen Signalkompressor (20) vor den Zwischenspeichern (5 bis 8) (Fig. 5).

18. Vorrichtung nach Anspruch 17, gekennzeichnet durch einen nach Wurzel- oder Logarithmusgesetz arbeitenden Kompressor (20).

19. Vorrichtung nach Anspruch 17 oder 18, gekennzeichnet durch einen Expander für die Echosignale, der dem Verhältnisbildner entweder vorgeschaltet oder bei einem durch einen zusätzlichen Wurzelbildner hindurchlaufenden Summensignal für den Verhältnisbildner demselben nachgeschaltet ist.

20. Vorrichtung nach Anspruch 19, gekennzeichnet durch einen nach quadratischem oder logarithmischem Gesetz arbeitenden Expander.

21. Vorrichtung nach einem der Ansprüche 17 bis 20, gekennzeichnet durch eine Serienschaltung für das jeweils abzubildende Echosignal $(x_p)$, bestehend aus einem Gleichrichter (22), einem Logarithmierglied (23) und einem Summen-Differenz-Bildner (21) mit einem ersten Summiereingang für das gleichgerichtete und logarithmierte Echosignal, einem zweiten Summiereingang für eine Rampenfunktion $(t/\tau)$ und mit einem Subtrahiereingang für das über ein Logarithmierglied (18) zugeführte Ausgangssignal der Speichereinheit (11) zur Verhältnisbildung.

## Claims

1. Process for the acquisition and recording of ultrasonic sectional images, wherein an object which is to be investigated is scanned row-wise by ultrasonic transmitted signals and the arising echo signals are likewise recorded row-wise on the recording carrier of an image recording device for the formation of an echo perspective image, characterised in that before the echo signals ($E_n$ to $E_{n+4}$) are forwarded to the image recording device (16), in the timing of the echo row incidence, successively a preselectable number (e. g. four) of rows ($Z_n$ to $Z_{n+3}$) which preferably, together with a current row ($Z_{n+4}$), determine the width of an outer field (U) for echoes (P) which are to be portrayed, is written into a correspondingly predetermined number of intermediate stores (5, 6, 7, 8) of a first storage unit which are subsequently scanned by a read-out pulse train which preferably corresponds to the write-in pulse train, and that the arising items of echo signal information are then fed to an analysis device (9 to 12) for purposes of analysis in that during a predetermined aperture time which determines the depth ($\tau$), extending in the row direction, of the outer field (U) for echoes (P) which are to be portrayed in the row field, at least approximately a mean value (S) of the echo intensity in the outer field is detected and — possibly following dynamic limitation, — is brought into ratio, in a ratio forming device (14), with the particular echo $(x_p)$ which is to be portrayed and whose output signal $(X_p)$ is the recording signal for the recording device (16), where the analysis device comprises an adder device (9) which is supplied with the items of echo signal information occurring in the intermediate stores (5 to 8) form equal depths of each scanning row, considered in the row direction, and which adds these items of signal information together with the associated current values of equal depth, and where these sum signals (s) at the output of the adder device (9) are added in a second storage unit (11) which also continuously stores the total sum of the sum signals (s) which is produced in this way, and simultaneously, for the step-wise forward pulsing of the outer field (U) for echoes which are to be portrayed in the row field, are also fed to a third storage unit (12), preferably a shift register, with a delay time $(\tau)$ which determines the depth of the outer field extending in the row direction, where the sum signals which occur in delayed fashion at the output of this third storage unit (12) are superimposed subtractively upon the storage signal of the second storage unit (11), which storage signal represents the total sum of the occurring sum signals.

2. Process as claimed in claim 1, characterised in that items of echo signal information from equal depths of each scanning row and stored by the intermediate stores (5 to 8) are scanned simultaneously and simultaneously forwarded to the adder device (9) for addition.

3. Process as claimed in claim 1 or 2, characterised in that the mean value of the echo intensity in the outer field is brought into ratio — possibly following dynamic limitation — in the ratio forming device (14) with a stored echo $(x_p)$ which is tapped from the intermediate stores (5 to 8) following a delay time which corresponds to half the number of stored rows of the outer field plus half the storage time of the further storage unit (11).

4. Process as claimed in one of the claims 1 to 3, characterised in that items of echo information arising from individual rows are continuously input into shift registers (5 to 8) which are assigned to the individual rows, are operated in series, and serve as intermediate stores, and are pulsed through said shift registers, and that the items of echo signal information which occur at the outputs of each shift register including the items of echo signal information which occur at the input of the first shift register are fed to the analysis device (9 to 12) in the order in which they occur.

5. Process as claimed in ohne of the claims 1 to 4, characterised in that the mean value signal (S) is fed to the ratio forming device (14) for dynamic compression via a root-forming device (13) (Fig. 1).

6. Process as claimed in one of the claims 1 to 5, characterised in that for the formation of the ratio (ratio forming device 14), following logarithmation in a logarithm forming device (18) the mean value signal (S) is fed, as control signal (y), to the control input for the degree of amplification of a control amplifier (17) with an exponential control curve featuring a negative exponent $(v_0 \cdot e^{-Ky})$ at the amplification input of which the particular echo signal which is to be portrayed is present (Fig. 4).

7. Process as claimed in claim 6, characterised in that the logarithmated mean value signal can be adjusted in arbitrary fashion in the amplification degree (A) by means of an interposed signal amplifier (19) so that for example, a ratio signal whose denominator value $(S^{KA})$ is determined by the set amplification degree (A) occurs at the output of the control amplifier (17).

8. Process as claimed in claim 7, characterised in that when the exponent is set at a value of 1/2, root dependency and thus halving of the dynamics is again achieved.

9. Process as claimed in one of the claims 1 to 4, characterised in that prior to intermediate storage in the intermediate stores (5 to 8) the original echo signal (x) is already compressed for the purpose of dynamic limitation (Fig. 5).

10. Process as claimed in claim 9, characterised in that the compression is carried out in accordance with a root function or logarithm function.

11. Process as claimed in claim 9 or claim 10, characterised in that echo signals which are to be portrayed prior to the ratio formation, or else the ratio signal itself are decompressed by expanders.

12. Process as claimed in claim 11, characterised in that the expansion is carried out in accordance with a quadratic or logarithmic law.

13. Process as claimed in one of the claims 9 to 12, characterised in that following rectification in a rectifier (22) the echo signals $(x_p)$ which are to be portrayed are logarithmated by means of a logarithmation element (23), and the logarithmated signal is fed to a first adder input of a sum-difference forming device (21) whose second adder input is supplied with a ramp function $(t/\tau)$ and whose subtractor input is fed with the logarithmic sum signal for the ratio formation (Fig. 5).

14. Device for the implementation of the process claimed in one or several of the claims 1 to 13, characterised by a predetermined number (e. g. four) of intermediate stores (5 to 8) into which the same number (here likewise four) of rows can be input in the timing of the echo row incidence in succession, and each of which stores one row, and characterised by a pulse generator (10) which is assigned to the intermediate stores (5 to 8) and which serves to read out the intermediately stored items of echo information in a predetermined read-out pulse train which preferably corresponds to the writein pulse train, and characterised by an analysis device (9 to 12) for the items of echo signal information at the output of the intermediate stores for the determination of a mean value (S), and characterised by a ratio forming device (14) which serves to form the ratio of an echo signal, which is to be portrayed, of the outer field and the mean signal value which prevails at the output of the analysis device and may previously have passed through a dynamic limiter (13), where the analysis device comprises an adder device (9), a second storage unit (11), and a third storage unit (12), of which the adder device (9) adds the items of echo signal information which each originate from the same row depth at the output of the intermediate stores (5 to 8) in order to form a mean value, and the second storage unit (11) represents a sample-and-hold circuit which adds all the sum signals from the adder device (9) in the pulse rate of the pulse generator (10), and where the output of the adder device (9) is connected directly to the adder input and via the third storage unit (12), preferably a shift register, which has a delay time $(\tau)$ which corresponds to the depth of the outer field, to a substractor input of the sample-and-hold circuit (11).

15. Device as claimed in claim 14, characterised by peak detectors (91) in the analysis device which serve to detect individual echo intensities.

16. Device as claimed in claim 14 or 15, characterised by a series arrangement of analogue shift registers (5 to 8) which serve as intermediate stores and which are connected at their inputs and outputs to inputs of the analysis device (9 to 12).

17. Device as claimed in one of the claims 14 to 16, characterised by a signal compressor (20) which precedes the intermediate stores (5 to 8) (Fig. 5).

18. Device as claimed in claim 17, characterised by a compressor (20) which operates in accordance with a root law or logarithm law.

19. Device as claimed in claim 17 or 18, characterised by an expander for the echo signals which either precedes the ratio forming device, or in the event of a sum signal for the ratio forming device which passes through an additional root forming device, is connected following the ratio forming device.

20. Device as claimed in claim 19, characterised by an expander which operates in accordance with a quadratic or logarithmic law.

21. Device as claimed in one of the claims 17 to 20, characterised by a series arrangement for the echo signal $(x_p)$ which is to be portrayed, composed of a rectifier (22), a logarithmation element (23), and a sum-difference forming device (21) which possesses a first adder input for the rectified and logarithmated echo signal, a second adder input for a ramp function $(t/\tau)$, and a subtractor input for the output signal of the storage unit (11), which has been conducted across a logarithmation element (18), for the ratio formation.

## Revendications

1. Procédé pour obtenir et tracer des tomographies par ultra-sons du type dans lequel l'objet à examiner est exploré ligne par ligne avec des signaux ultra-sonores d'émission, les signaux d'échos obtenus étant tracés de façon correspondante ligne par ligne sur un support d'enregistrement d'un dispositif d'enregistrement de l'image, sous la forme d'une image visible d'écho, caractérisé par le fait qu'avant la transmission des signaux d'échos $(E_n$ à $E_{n+4})$ au dispositif d'enregistrement de l'image (16), on procède, à la cadence de l'incidence des lignes d'échos et successivement dans le temps, à l'enregistrement ou inscription d'un nombre présélectionné de (par exemple quatre) lignes $(Z_n$ à $Z_{n+3})$ qui déterminent de préférence ensemble avec une ligne actuelle $(Z_{n+4})$ la largeur d'un champ ambiant (U) pour des échos à reproduire en image, dans un nombre correspondant et prédéterminé de mémoires intermédiaires (5, 6, 7, 8) d'une première unité de mémoires qui sont ensuite explorées à la cadence de lecture qui correspond de préférence à la cadence de l'inscription, et que des informations des signaux d'échos qui se présentent sont appliquées ensuite à un dispositif d'évaluation (9 à 12) pour leur évaluation dans un sens tel que pendant une durée prédéterminée la profondeur $(\tau)$ du champ ambiant (U), qui s'étend en direction des lignes et qui se fixe dans le champ des lignes pour des échos à reproduire en image, saisi dans le champ environnant au moins une valeur moyenne (S) de l'intensité de l'écho et est, éventuellement après une limitation dynamique, mis en rapport avec l'écho à reproduire $(x_p)$ dans un sommateur de rapport (14) dont le signal de sortie $(X_p)$ représente le signal à tracer pour le dispositif d'enregistrement (16) ce dernier comportant un dispositif de sommation (9) auquel sont appliqués les signaux d'information d'echos apparaissant au niveau des mémoires intermédiaires (5 à 8) et présentant, en direction des lignes, la même profondeur dans chaque ligne d'exploration, et qui additionne lesdites informations de signaux avec les valeurs actuelles associées de mêmes profondeurs et ces signaux de sommation (a) étant, au niveau de la sortie du dispositif de sommation (9), additionnés dans une seconde unité de mémoire (11) dans laquelle est également mémorisée de façon continue la somme totale des signaux de somme (s), et étant, en même temps, et pour le décalage cadencé du champ ambiant pour l'écho à reproduire dans le champ des lignes, appliqués à une troisième unité de mémoire (12) constituée de préférence par un registre à décalage, avec un retard $(\tau)$ qui détermine la profondeur du champ ambiant qui s'étend en direction des lignes, alors que les signaux de somme qui apparaissent avec retard à la sortie de cette troisième unité de mémoire (12) sont superposés, dans la seconde unité de mémoire (11), dans le sens d'une soustraction, au signal de mémoire représentant les signaux de somme.

2. Procédé selon la revendication 1, caractérisé par le fait que parmi des informations des signaux d'échos mémorisées dans les mémoires intermédiaires (5 à 8), sont explorées et simultanément transmises au dispositif de sommation (9) pour en établir la somme, celles qui possèdent la même profondeur dans chaque ligne d'exploration.

3. Procédé selon la revendication 1 ou 2, caractérisé par le fait que la valeur moyenne des intensités des échos dans le champ ambiant est, éventuellement après une limitation de dynamique antérieure, mise en rapport, dans un formateur de rapport (14), avec un écho de mémoire ($x_p$) prélevé dans les mémoires intermédiaires (5 à 8), après un retard temporel qui correspond à la moitié du nombre de lignes mémorisées du champ ambiant, augmentés de la moitié de la durée de mémorisation de la seconde unité de mémoire (11).

4. Procédé selon l'une des revendications 1 à 3, caractérisé par le fait que les informations d'échos disponibles pour les différentes lignes sont mémorisées de façon continue dans les registres à décalage (5 à 8) associés aux différentes lignes, montés en série et servant de mémoires intermédiaires, dans lesquelles lesdites informations sont décalées à la cadence appropriée et que les informations des signaux d'échos qui se présentent aux sorties de chacun des registres à décalage, y compris les informations de signaux d'échos qui sont disponibles à l'entrée du premier registre à décalage concerné, sont transmises en cadence aux dispositifs d'évaluation (9 à 12).

5. Procédé selon l'une des revendications 1 à 4, caractérisé par le fait que le signal de valeur moyenne (S) est appliqué au formateur de rapport (14) pour la compression de dynamique, par l'intermédiaire d'un formateur de radical (13) (fig. 1).

6. Procédé selon l'une des revendications 1 à 5, caractérisé par le fait que pour la formation du rapport (formateur de rapport 14) le signal de valeur moyenne (S) est appliqué, après formation du logarithme dans un formateur de logarithme (18) et sous la forme de signal de commande (y) à l'entrée de commande pour le coefficient d'amplification de l'amplificateur de commande (17) à caractéristique exponentielle à exposant négatif ($v_0 \cdot e^{-Ky}$), à l'entrée d'amplification duquel est appliqué le signal d'écho à reproduire (fig. 4).

7. Procédé selon la revendication 6, caractérisé par le fait que le logarithme du signal de valeur moyenne est ajusté à volonté par un amplificateur de signaux intermédiaires (19), du point de vue de son degré d'amplification (A), et cela de manière que l'on obtienne, par exemple, à la sortie de l'amplificateur de commande (17) un signal de rapport dont le numérateur ($S^{KA}$) est déterminé par le degré d'amplification (A) qui se règle.

8. Procédé selon la revendication 7, caractérisé par le fait que lors du réglage de l'exposant à la valeur 1/2 on obtient à nouveau une dépendance radicale et, par voie de conséquence, une division par deux de la dynamique.

9. Procédé selon l'une des revendications 1 à 4, caractérisé par le fait que le signal d'échos originel (x) est, avant mémorisation dans les mémoires intermédiaires (5 à 8), comprimé en vue de la limitation de dynamique (fig. 5).

10. Procédé selon la revendication 9, caractérisé par le fait que la compression est opérée suivant une fonction radicale ou logarithmique.

11. Procédé selon la revendication 9 ou 10, caractérisé par le fait que les signaux d'échos à reproduire sont décomprimés avant formation du rapport, à l'aide d'un expanseur ou que le signal de rapport est décomprimélui-même à d'aide d'un expanseur.

12. Procédé selon la revendication 11, caractérisé par le fait que l'expansion est opérée suivant une loi quadratique ou logarithmique.

13. Procédé selon l'une des revendications 9 à 12, caractérisé par le fait que les signaux d'échos à reproduire ($x_p$) sont, après redressement dans un redresseur (22) transformés en logarithmes à l'aide d'un circuit de formation de logarithmes (23) et que le signal logarithmique est appliqué à une première entrée de sommation d'un formateur somme-différence (21) dont la seconde entrée de sommation est chargée avec une fonction-rampe ($t/\tau$) alors que son entrée de soustraction est chargée avec le signal somme logarithmique pour la formation du rapport (fig. 5).

14. Dispositif pour la mise en oeuvre du procédé selon une ou plusieurs des revendications 1 à 13, caractérisé par le fait qu'il comporte un nombre prédéterminé (par exemple quatre) de mémoires intermédiaires (5 à 8) dans lesquelles est susceptible d'être inscrite, à la cadence des lignes d'échos qui se présentent et successivement dans le temps, respectivement le même nombre (ici également de quatre) lignes et dont chacun mémorise une ligne, un générateur de cadence (10) associé aux mémoires intermédiaires (5 à 8) pour lire les informations d'échos mémorisées temporairement, la lecture se faisant à une cadence donnée à l'avance qui correspond de préférence à la cadence d'inscription, un dispositif d'évaluation (9 à 12) pour les informations des signaux d'échos au niveau de la sortie des mémoires intermédiaires en vue de déterminer une valeur moyenne (s), et un formateur de rapport (14) pour former le rapport entre un signal d'écho, à reproduire, du champ environnant et le signal de valeurs moyennes à la sortie du dispositif d'évaluation, qui a été précédemment amené à traverser un limiteur de dynamique (13), ledit dispositif d'évaluation comprenant un dispositif de sommation (9) ainsi qu'une seconde unité de mémorisation (11) et une troisième unité de mémorisation (12) parmi lesquelles l'unité de sommation (9) fait la somme des informations de signaux d'échos de même profondeurs de lignes à la sortie des mémoires intermédiaires (5 à 8) pour la formation d'une valeur moyenne, alors que la seconde unité de mémorisation (11) est constituée par un circuit de maintien dy type sample-and-hold qui additionne tous les signaux somme de l'unité de sommation (9) à la cadence du génératuer de cadence (10) et que la sortie du dispositif de sommation (9) est reliée directement à l'entrée de sommation du circuit de commutation sample-and-hold (11) et indirectement, et par l'intermédiaire de la troisième unité de mémorisation (12) qui est constituée de préférence par un registre à décalage possédant une durée de retard ($\tau$) qui correspond à la profondeur du champ ambiant, avec l'entrée de soustraction dudit dispositif de commutation (11).

15. Dispositif selon la revendication 14, caractérisé par le fait qu'il comporte des détecteurs de valeurs de pointe dans le dispositif d'évaluation pour saisir différentes intensités d'échos.

16. Dispositif selon la revendication 14 ou 15, caractérisé par un montage série constitué par des registres à décalage analogiques (5 à 8) comme mémoires intermédiaires lesquels registres sont reliés, du côté entrée et du côté sortie, avec des entrées du dispositif d'évaluation.

17. Dispositif selon l'une des revendications 14 à 16, caractérisé par un compresseur de signaux (20) disposé en amont des mémoires intermédiaires (5 à 8) (fig. 8).

18. Dispositif selon la revendication 17, caractérisé par un compresseur (20) opérant suivant une loi radicale ou logarithmique.

19. Dispositif selon la revendication 17 ou 18, caractérisé par un expanseur pour les signaux d'échos, qui est monté soit en amont du formateur de rapport, soit en aval de ce dernier dans le cas d'un signal somme, pour le formateur de rapport qui passe par un formateur radical supplémentaire.

20. Dispositif selon la revendication 19, caractérisé par un expanseur opérant selon une loi quadratique ou logarithmique.

21. Dispositif selon l'une des revendications 17 à 20, caractérisé par un montage série pour le signal d'écho ($x_p$) à reproduire, constitué par un redresseur (22), par un circuit de formation du logarithme (23) et par un formateur somme-différence (21) comportant une première entrée de sommation pour le signal d'écho redressé et dont le logarithme a été établi, une seconde entrée de sommation pour une fonction-rampe ($t/\tau$) et une entrée de soustraction pour le signal de sortie de l'unité de mémorisation (11) appliqué par l'intermédiaire d'un dispositif de formation du logarithme (18), en vue de la formation du rapport.

FIG 3

$x$

$Z_{n+4}$   R   $Z_{n+3}$   R   $Z_{n+2}$   P   R   $Z_{n+1}$   R   $Z_n$

$E_{n+4}$   $x_{i,n+4}$   $E_{n+3}$   $x_{i,n+3}$   $E_{n+2}$   $x_{i,n+2}$   $E_{n+1}$   $x_{i,n+1}$   $E_n$   $x_{i,n}$   $t$

1

4   8   7   6   5

2

$\frac{\tau}{2}$   15

3

9

$s$

$\tau$   12

$x_p$

P   $\tau$   Z

$n$
$n+1$
$n+2$
$n+3$
$n+4$

U   U   P

$x_{1,n} \cdots x_{1,n+4}$

$x_{j,n} \cdots x_{j,n+4}$

10   +   11   −   S   $\sqrt{S}$   13   $\frac{x_p}{\sqrt{S}}$   14   $x_p$   16

FIG 2     FIG 1

FIG 4

FIG 5